# EUROPEAN PATENT SPECIFICATION

(11) **EP 1 647 556 B1**
(45) Date of publication and mention of the grant of the patent: **19.05.2010**
(21) Application number: 04024507.8
(22) Date of filing: 14.10.2004
(51) Int. Cl.: C07K 7/08, A61K 38/08, G01N 33/00, A61K 38/04, A61K 38/10

(54) **Peptidic compounds and derivatives thereof for the treatment of human diseases through inhibition of signaling via growth factors**
Peptidische Verbindungen und deren Ableitungen für die Behandllung von menschlichen Krankheiten durch Hemmung der Signalübertragung über Wachstumfaktoren
Composés et dérivés peptidiques pour le traitement de maladies humaines par inhibition de la transmission du signal par des facteurs de croissances

(43) Date of publication of application: 19.04.2006
(73) Proprietor: Karlsruher Institut für Technologie, 76131 Karlsruhe (DE)
(72) Inventor: Hentsch, Bernd, 81371 München (DE); Ponta, Helmut, 76356 Weingarten (DE); Herrlich, Peter A., 76229 Karlsruhe (DE); Matzke, Alexandra, 75179 Pforzheim (DE); Orian-Rousseau, Veronique, 67690 Rittershoffen (FR); Adam, Jan, 76133 Karlsruhe (DE)
(74) Representative: Keller, Günter

(56) References cited:
- EP-A1- 1 391 213
- US-A1- 2003 069 181
- ORIAN-ROUSSEAU VÉRONIQUE ET AL: "CD44 is required for two consecutive steps in HGF/c-Met signaling." GENES & DEVELOPMENT. 1 DEC 2002, vol. 16, no. 23, 1 December 2002 (2002-12-01), pages 3074-3086, XP002326889 ISSN: 0890-9369
- LACKNER CAROLIN ET AL: "Soluble CD44 v5 and v6 in serum of patients with breast cancer. Correlation with expression of CD44 v5 and v6 variants in primary tumors and location of distant metastasis" BREAST CANCER RESEARCH AND TREATMENT, vol. 47, no. 1, January 1998 (1998-01), pages 29-40, XP002326890 ISSN: 0167-6806
- A. MATZKE, ET AL.: "A five-amino acid peptide blocks Met and Ron-dependent cell migration" CANCER RESEARCH, vol. 64, no. 14, 15 July 2005 (2005-07-15), pages 6105-6110,

## Description

The present invention provides peptide compounds designed to deliver growth factor receptor inhibitory activity. The invention relates to the use of such peptide drugs for the treatment of human diseases through the inhibition of growth factor receptor signaling, in particular involving signals mediated by hepatocyte growth factor (HGF), c-met and CD44. The invention comprises a use of compounds for the manufacture of a medicament for the treatment and prevention of hyperproliferative disorders or diseases associated with an increased invasive potential of cells, such as cancer, including the treatment and prevention of tumor metastases and minimal residual disease. The invention includes the manufacture of a clinically used medicament for the treatment and prevention of human diseases associated with abberant gene expression patterns due to aberrant signaling via HGF.

### Summary

The tyrosine kinase receptor c-Met and its ligand HGF/SF (hepatocyte growth factor/scatter factor), ezrin and splice variants of CD44 have independently been identified as tumor metastasis-associated proteins. It has been shown recently that these proteins cooperate and that a CD44 isoform containing variant exon v6 sequences is strictly required for c-Met activation by HGF/SF in rat and human carcinoma cells, in established cell lines as well as in primary keratinocytes. CD44 v6 deficient tumor cells were unable to activate c-Met unless they were transfected with a CD44 v6-bearing isoform. Antibodies to v6-encoded epitopes inhibited autophosphorylation of c-Met by interfering with the formation of a complex formed by c-Met, CD44 v6 and HGF/SF. In addition, signal transduction from activated c-Met to MEK and Erk required presence of the cytoplasmic tail of CD44 including a binding motif for ERM proteins (Orian-Rousseau V, Chen L, Sleeman JP, Herrlich P, Ponta H, Genes Dev. 2002 Dec 1;16(23):3074-86). This suggests a role for ERM proteins and possibly their link to the cortical actin cytoskeleton in signal transfer.

The present invention describes the identification of short peptide motifs, derived from the CD44 v6 region, which are capable of interfering with HGF signaling via inhibition of the formation of the trimeric complex made up by c-Met, HGF and CD44. Methods for using such peptides and compositions for modulating the formation of a complex made up by c-Met, HGF and CD44 in a variety of contexts are also provided. This invention is furthermore useful for the development of peptide drugs or chemical compounds structurally derived therefrom, that interfere with signaling of HGF and/or the formation of this trimeric complex. Such drugs might be therapeutically useful for diseases in which the inhibition of such an interaction of c-Met, HGF and CD44 has a beneficial effect. Such indications include, but are not limited to, hyperproliferative diseases like cancer or diseases associated with invasive processes of cells, such as macrophages, lymphocytes and/or granulocytes.

### Introduction

The receptor tyrosine kinase c-Met (here designated Met) and its ligand hepatocyte growth factor/scatter factor HGF/SF (here designated HGF) control several cellular processes essential for life. Disruption of their genes in the mouse causes embryonic midgestation death due to placental failure and reduced development of several epithelial organs (reviewed by Birchmeier and Gherardi, 1998, Trends Cell Biol 8, 404-10). Analyses of these mice and studies with organ and cell cultures revealed roles for HGF/Met in invasive growth and cellular migration, in proliferation and differentiation, in mesenchymal-epithelial communication as well as in tubular epithelial organisation and morphogenesis (reviewed by Bardelli and Comoglio, 1997, Ciba Found Symp 212, 133-44; Birchmeier and Gherardi, 1998, Trends Cell Biol 8, 404-10). Met is predominantly expressed in epithelial cells while HGF is secreted by mesenchymal cells. HGF can bind to heparan sulfate proteoglycans (HSPGs) (Mizuno et al., 1994, J Biol Chem 269, 1131-6), but it is controversial whether the function of HSPG-binding is needed for the signaling process. In CD44-transfected Namalwa cells, HGF binding to a CD44HSPG enhanced Met activation (van der Voort et al., 1999, J. Biol. Chem. 274, 6499-6506), while in MDCK and mink lung cells, a HGF mutant which cannot bind to heparan sulfate (HS), activated Met just like the wild type HGF (Hartmann et al., 1998, Curr Biol 8, 125-34).

Cancer cells subvert the HGF/Met system. In oncogenic Tpr-Met, for instance, the "negatively acting membrane-proximal domain" of Met is replaced by the dimerization helix of Tpr thus causing ligand-independent Met activation and oncogenesis (Rodrigues and Park, 1993, Mol Cell Biol 13, 6711-22; Vigna et al., 1999, Oncogene 18, 4275-81). Met promotes the invasive and metastatic properties of tumor cells in several animal systems (Giordano et al., 1993, Proc Natl Acad Sci U S A 90, 649-53; Rong et al., 1994, Proc Natl Acad Sci U S A 91, 4731-5). In various human tumors gene amplification of met, upregulation of Met expression and mutations in met have been detected (reviewed by Bardelli et al., 1997, Biochim Biophys Acta 1333, M41-51).

Invasive and metastatic growth is not only influenced by HGF/Met. Other growth factors, the extracellular matrix, the functional state of adhesion molecules and of their intracellular complexes, are also important determinants (Habets et al., 1994, Cell 77, 537-49; Camenisch et al., 2000; Hobson et al., 2001, Science 291, 1800-3). Genome-wide unbiased screens, e.g., by suppressive subtractive hybridization, identified numerous metastasis-associated genes (Nestl et al., 2001, Cancer Res 61, 1569-77). Interestingly Met, ezrin, metalloproteases (MMPs) and urokinase plasminogen activator (uPA) receptor were found overexpressed in several types of metastatic cancer cells.

One of the earliest screens for metastasis-associated genes identified variant isoforms of CD44 (Günthert et al., 1991, Cell 65, 13-24). The designation CD44 describes a family of class I transmembrane proteins produced by extensive alternative splicing. The variation is predominantly in the extracellular membrane-proximal portion of the proteins encoded by variant exons v1 to v10. An additional degree of variability of CD44 is introduced by posttranslational modifications (reviewed by Naor et al., 1997, in Advances in Cancer Research, G. F. Vande Woude and G. Klein, eds., San-Diego, pp. 243-318; Ponta and Herrlich, 1998, Frontiers in Biosc. 3, d650-d656).

A causal role for CD44 isoforms in the formation of metastases has been documented by ectopic expression in certain non-metastatic cell lines. Isoforms bearing sequences encoded by exons v4 through v7 or v6 plus v7 (CD44v4-7 and CD44v6,7 respectively) sufficed to confer metastatic potential to these cells (Gunthert et al., 1991, Cell 65, 13-24; Rudy et al., 1993, Cancer Res 53, 1262-8). Antibodies directed against a v6-encoded epitope or CD44 v6 antisense abrogated tumor growth and metastatic outgrowth in vivo and invasiveness of fibrosarcoma cells in vitro (reviewed by Naor et al., 1997, in Advances in Cancer Research, G. F. Vande Woude and G. Klein, eds., San-Diego, pp. 243-318; Ponta and Herrlich, 1998, Frontiers in Biosc. 3, d650-d656).

CD44 null mice are viable and show very circumscribed deficiencies, for example of the immune system and of cell migration in the embryo (Schmits et al., 1997, Blood 90, 2217-2233; Protin et al., 1999, J. Immunol. 163, 4917-4923; see also the discussion section in Camenisch et al., 2000, J Clin Invest 106, 349-360). The current hypothesis is that efficient substitute molecules take over CD44 functions in early embryonic development but cannot be established after differentiation. This notion is supported by the observations that perinatal antisense CD44 expression in keratinocytes or antibody interference with CD44 function in the differentiated cells of the apical ectodermal ridge during limb development caused severe defects related to inhibited growth factor function (Kaya et al., 1997, Genes Dev 11, 996-1007; Sherman et al., 1998, Genes Dev. 12, 1058-1071). As a possible mechanism for the action of CD44, the sequestering and "presentation" of growth factors by CD44 has previously been suggested (Tanaka et al., 1993, Nature 361, 79-82; Bennett et al., 1995, J. Cell Biol. 128, 687-698). Several growth factors, FGFs as prime examples, bind to heparan sulfate (HS) and their receptors require HS association for signaling (Schlessinger et al., 1995, Cell 83, 357-60.; Plotnikov et al., 1999, Cell 98, 641-50). HS can indeed be covalently linked to CD44, predominantly or exclusively to a sequence motif encoded by exon v3. CD44v3 could thus function in FGF "presentation" (Sherman et al., 1998, Genes Dev. 12, 1058-1071).

The upregulation of CD44 variant proteins and of Met in cancer as well as possible roles for both types of molecules in metastatic growth and invasion lead to the examination of a possible molecular interplay between them. In several types of cells including primary keratinocytes, HGF-induced Met activation and signaling depends absolutely on the presence of CD44 isoforms bearing the v6 sequence. HS-modification of CD44 is not required for this synergy. Mature HGF, Met and v6-containing CD44 proteins form a complex. Met activation as well as complex formation is prevented by antibodies recognizing the v6 epitope or other epitopes in the membrane-proximal stem structure of CD44. Interestingly, CD44 v6-containing isoforms catalyze two distinct and separable steps during HGF-dependent Met signaling. For Met autophosphorylation the extracellular domain of CD44 is required and sufficient. Transfer of the signal from activated Met to MEK and Erk depends, however, on the presence of the cytoplasmic tail of CD44 and most likely its associated actin-binding protein ezrin suggesting a role of the cytoskeletal organization in signal transduction.

Invasiveness and metastatic migration of cancer cells are in part regulated by the receptor tyrosine kinase Met. Met can be activated by mutation, but most frequently depends on stimulation by its ligand HGF produced either by the tumor cells or by stromal cells (reviewed in Bardelli et al., 1997, Biochim Biophys Acta 1333, M41-51). In several different transformed and non-transformed cells activation of Met by its authentic ligand HGF depends strictly on the function of CD44 isoforms that carry the exon v6encoded protein sequence. The contribution of CD44 is two-fold: The extracellular domain of CD44 with the v6 sequence is required to organize a ternary complex between Met, HGF and CD44 which is a prerequisite of Met activation. The cytoplasmic tail of the v6-containing CD44 isoform assembles protein partners necessary for signal transfer from Met to MEK and Erk. Sequestration experiments using soluble cytoplasmic tail peptides indicated that ERM proteins are necessary as protein partners in the signaling process (ezrin is the major ERM member in tumor cells). These data thus identify a mechanistic link between four molecules that have been implicated in tumor metastasis formation: HGF/Met (reviewed in Bardelli et al., 1997, Biochim Biophys Acta 1333, M41-51), CD44 (reviewed in Naor et al., 1997, in Advances in Cancer Research, G. F. Vande Woude and G. Klein, eds., San-Diego, pp. 243-318) and ezrin (Fazioli et al., 1993, Oncogene 8, 1335-45).

How can the extracellular domain of CD44 variant proteins support Met activation? It has been reported previously that HS-binding growth factors such as FGFs profit from the presence of large CD44 splice forms which act as HSPGs (Bennett et al., 1995, J. Cell Biol. 128, 687-698 and J. Cell Biol. 131, 1623-1633; Sherman et al., 1998, Genes Dev. 12, 1058-1071). The v3 sequence carries the unique HS-addition site of CD44 and was required for FGF action. HGF can also bind to HS and a supportive effect of CD44HSPG on Met activation has been found on Namalwa cells (van der Voort et al., 1999, J. Biol. Chem. 274, 6499-6506). An HGF mutant which cannot bind to HS was, however, not defective in its Met-activating potential but rather exhibited enhanced activity (Hartmann et al., 1998, Curr Biol 8, 125-34). Also in cell lines, neither the v3 exon-associated HS nor any other HSPG was needed for CD44-dependent Met activation in response to added mature HGF.

As an activation of the HGF-proform by CD44 has been excluded, two interpretations remain to explain the role of the v6-dependent clustering in the activation of Met. Either HGF requires CD44 help to bind to Met, or v6-bearing CD44 prevents access of a negatively-acting component to Met. Negative components acting on Met could be specific transmembrane or cytosolic protein tyrosine phosphatases interacting with Met (Kulas et al., 1996, J Biol Chem 271, 748-54; Villa-Moruzzi et al., 1998, Biochem J 336, 235-9). Indeed, negative control appears to be eliminated in constitutivly active oncogenic Tpr-Met where the extracellular domain of Met and an intracellular "negative domain" are lost (Vigna et al., 1999, Oncogene 18, 4275-81). Currently the interpretation that a negative regulator is excluded appears favorable. Apparently, for this function CD44 needs to be anchored in the plasma membrane since a soluble CD44 v6 containing isoform does not support Met activation but rather inhibits.

The close proximity of Met and CD44 mediated by v6 appears, in addtion, to be important for a second step of synergy absolutely required for HGF-induced signaling. The cytoplasmic tail of CD44 potentiates signal transduction from the multimeric complex. Recent data suggest that an essential partner protein is ezrin (or another member of the ERM family), although other proteins with similar binding preference are not strictly ruled out. Ezrin can link the actin cytoskeleton to the plasma membrane through binding of its C-terminus to F-actin, and of its N-terminus to CD44 (reviewed in Tsukita and Yonemura, 1999, J Biol Chem 274, 34507-10). For this function, ezrin needs to be phosphorylated which, interestingly, can also occur in a Met-dependent reaction (Crepaldi et al., 1997, J Cell Biol 138, 423-34). Lack of the cytoplasmic tail of CD44 or the sequestering of ezrin away from its location by excess soluble CD44 tails in the cytoplasm caused block of HGF-induced signal transfer as well as disruption of cortical actin. This finding may be interpreted to indicate a need for structural organization of the cytoskeleton in the immediate proximity of Met which is mediated by ezrin. The CD44v6 isoform focusses the signaling components specifically to Met. Interestingly, replacement of ezrin by the tumor suppressor protein merlin (Morrison et al., 2001, Genes Dev 15, 968-80) also caused disruption of cortical actin structure and a block of signal transduction (H. Morrison, H. Ponta and P. Herrlich, unpublished data).

The idea that a strict structural organization at the inner side of the plasma membrane controls signal transduction is supported by several other observations. The presence of α-catenin bound to E-cadherin appears to exert a negative control on signal transduction downstream of receptor tyrosine kinases, but upstream of Ras (Vasioukhin et al., 2001, Cell 104, 605-17) and α-catenin links E-cadherin to F-actin. Ankyrin which also bridges CD44 to the cytoskeleton, was found to recruit Src into the complex (Bourguignon et al., 2001, J Biol Chem 276, 7327-36). The view that signaling components are strictly organised in a preformed order as modules, emerges from various data, e.g. the existence of scaffolding proteins and the confined localization of c-AMP, PKA and phosphodiesterase (Whitmarsh et al., 1998, Science 281, 1671-4; Yasuda et al., 1999, Mol Cell Biol 19, 7245-54).

CD44 was originally identified as an adhesion molecule that binds to ECM components (reviewed in Naor et al., 1997, in Advances in Cancer Research, G. F. Vande Woude and G. Klein, eds. San-Diego, pp. 243-318; Ponta et al., 1998, Frontiers in Biosc. 3, d650-d656). In addition to the synergizing pair CD44/Met, other receptor tyrosine kinases are associated with adhesion molecules. Interactions have been described between ErbB2 and CD44s (Bourguignon et al., 1997, J Biol Chem 272, 27913-8; Sherman et al., 2000, J Cell Biol 150, 1071-84), and between FGF receptor 4, N-cadherin and N-CAM (Cavallaro et al., 2001, Nat Cell Biol 3, 650-7.). Intriguingly, also CD44 and integrins seem to be linked in that they coordinately affect the binding to cells of another invasiveness-associated molecule, osteopontin, and in that cross-linking of CD44 upregulates integrin activity (Fujisaki et al., 1999, Cancer Res. 59, 4427-4434; Katagiri et al., 1999, Cancer Res 59, 219-26). In turn, the activation of Met upregulates expression of osteopontin (Medico et al., 2001, Cancer Res 61, 5861-8), presumably increasing expression of CD44 and perhaps even regulating alternative splicing through activation of Ras (Hofmann et al., 1993, Cancer Res 53, 1516-21; König et al., 1998, EMBO J. 10, 2904-2913). These examples suggest the existence of a network of protein complexes in the plasma membrane and of several regulatory loops. Interestingly, in cells lacking the integrin subunit β4 Met could not be activated (Trusolino et al., 2001, Cell 107, 643-54). Met signaling could be re-established by introduction of β4 which was found to associate with Met. The possibility thus exists that α6β4 integrin, CD44 and Met are located in one and the same membrane domain.

Why should the activation of a growth factor receptor depend on a cooperating transmembrane protein? A plausible explanation was recently presented by the finding that CD44 monitors the cellular environment through its N-terminal domain (Morrison et al., 2001, Genes Dev 15, 968-80). Cell-cell contact and the presence of high molecular weight hyaluronate affected the activity state of CD44 which resulted in a change in intracellular partner proteins and had a dramatic effect on signal transduction and gene expression (H. Morrison, H. Ponta and P. Herrlich, unpublished data). The formation of multicomponent complexes at the cell surface, involving, e.g., such a complex composed of Met, HGF and CD44 as described in this invention, could be the first decisive step that determines the programs leading to proliferation, migration, invasiveness or cell cycle arrest.

Thus, the disruption of such complexes or the inhibition of the formation of such complexes could in fact serve as a therapeutic strategy to interfere with processes leading to human diseases, such as cancer and to tumor metastases formation.

The present invention describes compounds that display this activity, namely the inhibition of the complex formation consisting of Met, HGF and CD44. Therefore, these compounds may be useful for the treatment of diseases in which the inhibition of such a complex has a beneficial effect. Such indications include, but are not limited to, hyperproliferative diseases like cancer or diseases associated with invasive processes of cells, such as macrophages, lymphocytes and/or granulocytes.

The invention relates to a peptide compound selected from the group consisting of
(a) peptides consisting of the amino acid sequence as shown in SEQ ID NO:1 or SEQ ID NO:2;
(b) peptides consisting of any one of the following amino acid sequences:
   amino acids 2-14 of SEQ ID NO:1 or 2;
   amino acids 2-13 of SEQ ID NO:1 or 2;
   amino acids 2-12 of SEQ ID NO:1 or 2;
   amino acids 2-11 of SEQ ID NO: 1 or 2;
   amino acids 3-14 of SEQ ID NO:1 or 2;
   amino acids 3-13 of SEQ ID NO:1 or 2;
   amino acids 3-12 of SEQ ID NO:1 or 2;
   amino acids 3-11 of SEQ ID NO:1 or 2;
   amino acids 4-14 of SEQ ID NO:1;
   amino acids 4-13 of SEQ ID NO:1 or 2;
   amino acids 4-12 of SEQ ID NO:1 or2;
   amino acids 4-11 of SEQ ID NO: 1 or 2;
   amino acids 5-14 of SEQ ID NO: 1 or 2;
   amino acids 5-13 of SEQ ID NO:1 or 2;
   amino acids 5-12 of SEQ ID NO:1 or 2;
   amino acids 5-11 of SEQ ID NO:1 or 2;
   amino acids 6-14 of SEQ ID NO:1 or 2;
   amino acids 6-13 of SEQ ID NO:1 or 2;
   amino acids 6-12 of SEQ ID NO:1 or 2;
   amino acids 6-11 of SEQ ID NO:1 or 2;
   amino acids 7-14 of SEQ ID NO:1 or 2;
   amino acids 7-13 of SEQ ID NO:1 or 2;
   amino acids 7-12 of SEQ ID NO:1 or 2;
   amino acids 7-11 of SEQ ID NO:1 or 2;
   and
(c) heterologous fusion peptides consisting of (a) or (b) fused to a heterologous amino acid sequence.

The term "peptide" as used herein refers to any compound comprising two or more amino acids joined to each other by peptide bonds or modified peptide bonds, i.e., peptide isosteres. "Peptide" refers to both short chains and to longer chains, generally referred to as polypeptides. Peptides may contain amino acids other than the 20 gene-encoded amino acids. "Peptides" include amino acid sequences modified either by natural processes, such as post-translational processing, or by chemical modification techniques which are well known in the art. Such modifications are well described in basic texts and in more detailed monographs, as well as in the research literature. Modifications can occur anywhere in a peptide, including the peptide backbone, the amino acid side-chains and the amino or carboxyl termini. It will be appreciated that the same type of modification may be present in the same or varying degrees at several sites in a given peptide. Also, a given peptide may contain many types of modifications.

Peptides may be branched and they may be cyclic, with or without branching. Cyclic, branched and branched cyclic peptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, ubiquitination and sumoylation.

The term "peptide compound" includes salts, preferably pharmaceutically acceptable salts of the peptides described herein. Salts encompassed within the term "pharmaceutically acceptable salts" refer to non-toxic salts of the peptide compounds of this invention. Representative salts and esters include the following: acetate, ascorbate, benzenesulfonate, benzoate, bicarbonate, bisulfate, bitartrate, borate, caamsylate, carbonate, citrate, dihydrochloride, methanesulfonate, ethanesulfonate, p-toluenesulfonate, cyclohexylsulfamate, quinate, edetate, edisylate, estolate, esylate, fumarate, gluconate, glutamate, glycerophophates, hydrobromide, hydrochloride, hydroxynaphthoate, lactate, lactobionate, laurate, malate, maleate, mandelate, mesylate, mucate, napsylate, nitrate, n-methylglucamine, oleate, oxalate, palmoates, pamoate (embonate), palmitate, pantothenate, perchlorates, phosphate/diphosphate, polygalacturonate, salicylates, stearate, succinates, sulfate, sulfamate, subacetate, succinate, tannate, tartrate, tosylate, trifluoroacetate, and valerate. Other salts include Ca, Li, Mg, Na, and K salts; salts of amino acids such as lysine or arginine; guanidine, diethanolamine or choline; ammonium, substituted ammonium salts or aluminum salts. The salts are prepared by conventional methods.

The peptide of the invention has a length of at least 2 amino acids. Preferably, the length of the peptide is at least 4, more preferably at least 6, more preferably at least 8, most preferably at least 10 amino acids. The maximum length is not particularly limited. It is preferred, however, that the peptide has a length of from about 6 to about 30 amino acids, preferably of from about 8 to about 25 amino acids, more preferably of from about 10 to about 20 amino acids, most preferably of from about 10 to about 15 amino acids. Larger peptides may be employed, for example when fusion peptides with heterologous amino acid sequences are prepared.
The term "peptide compound" as used herein denotes a compound comprising a peptide. The term "peptide compound" includes compounds comprising a peptide and a chemical moiety which is not an amino acid.

In a first embodiment, the peptide compound of the invention is a peptide consisting of the amino acid sequence as shown in SEQ ID NO:1 or 2 with SEQ ID NO:2 being preferred.

Preferably, the peptide compound may consist of a fragment of SEQ ID NO:3 or 4, said fragment having an activity of inhibiting the complex formation between Met, HGF and CD44 leading to an inhibition of HGF mediated activation of ERK. Preferably, the peptide compound consists of a fragment of SEQ ID NO:4, even more preferably of a fragment of SEQ ID NO: 2.

The term "fragment" of a given peptide is meant to refer to any peptide subset of said peptide. Generally, a fragment consists of at least 2 contiguous amino acids of the sequence of said peptide. Preferably, the fragment consists of at least 4, more preferably of at least 6, more preferably of at least 8, even more prefer ably of at least 10 contiguous amino acids of said peptide.

The "activity of inhibiting the complex formation between Met, HGF and CD44 leading to an inhibition of HGF mediated activation of ERK " can be determined in accordance with the method described in Examples 3 and 4 of this application. Usually, the peptide compound of the invention has the activity of inhibiting the complex formation between Met, HGF and CD44 leading to an inhibition of HGF mediated activation of ERK, measured in form of the activation of the ERK protein through phosphorylation. The inhibitory activity results in a reduction of downstream ERK activation. Preferably, downstream ERK activation is reduced by at least 30%, more preferably by at least 50% and even more preferably by at least 70% with respect to the downstream ERK activation in the presence of a control peptide (e.g. SEQ ID NO:8). Preferably, the activity is determined in cell culture.

In yet another aspect, the peptide compound may consist of a fragment of SEQ ID NO:1 or 2, said peptide compound having the activity of inhibiting the complex formation between Met, HGF and CD44 leading to an inhibition of HGF mediated activation of ERK. Preferably, the peptide compound consists of a fragment of SEQ ID NO:2.

Further described herein is a peptide compound comprising one of the following CD44-derived amino acid sequences:
amino acids 1-14 of SEQ ID NO:1 or 2;
amino acids 2-14 of SEQ ID NO:1 or 2;
amino acids 2-13 of SEQ ID NO:1 or 2;
amino acids 2-12 of SEQ ID NO:1 or 2;
amino acids 2-11 of SEQ ID NO:1 or 2;
amino acids 2-10 of SEQ ID NO:1 or 2;
amino acids 2-9 of SEQ ID NO:1 or 2;
amino acids 2-8 of SEQ ID NO:1 or 2;
amino acids 3-14 of SEQ ID NO:1 or 2;
amino acids 3-13 of SEQ ID NO:1 or 2;
amino acids 3-12 of SEQ ID NO:1 or 2;
amino acids 3-11 of SEQ ID NO:1 or 2;
amino acids 3-10 of SEQ ID NO:1 or 2;
amino acids 3-9 of SEQ ID NO:1 or 2;
amino acids 3-8 of SEQ ID NO:1 or 2;
amino acids 4-14 of SEQ ID NO:1 or 2;
amino acids 4-13 of SEQ ID NO:1 or 2;
amino acids 4-12 of SEQ ID NO:1 or 2;
amino acids 4-11 of SEQ ID NO:1 or 2;
amino acids 4-10 of SEQ ID NO:1 or 2;
amino acids 4-9 of SEQ ID NO:1 or 2;
amino acids 4-8 of SEQ ID NO:1 or 2;
amino acids 5-14 of SEQ ID N0:1 or 2;
amino acids 5-13 of SEQ ID NO:1 or 2;
amino acids 5-12 of SEQ ID NO:1 or 2;
amino acids 5-11 of SEQ ID NO:1 or 2;
amino acids 5-10 of SEQ ID NO:1 or 2;
amino acids 5-9 of SEQ ID NO: 1 or 2;
amino acids 5-8 of SEQ ID NO:1 or 2;
amino acids 6-14 of SEQ ID NO:1 or 2;
amino acids 6-13 of SEQ ID NO:1 or 2;
amino acids 6-12 of SEQ ID NO:1 or 2;
amino acids 6-11 of SEQ ID NO:1 or 2;
amino acids 6-10 of SEQ ID NO:1 or 2;
amino acids 6-9 of SEQ ID NO:1 or 2;
amino acids 6-8 of SEQ ID NO:1 or 2;
amino acids 7-14 of SEQ ID NO:1 or 2;
amino acids 7-13 of SEQ ID NO:1 or 2;
amino acids 7-12 of SEQ ID NO:1 or 2;
amino acids 7-11 of SEQ ID NO:1 or 2;
amino acids 7-10 of SEQ ID NO:1 or 2;
amino acids 7-9 of SEQ ID NO:1 or 2; or
amino acids 7-8 of SEQ ID NO:1 or 2.

These amino acid sequences correspond to those shown in table 1 and 2 infra.

In a specific embodiment, the CD44-derived amino acid sequence in the peptide compound of the invention is limited to any one of these amino acid sequences. That means, the peptide compound does not comprise other amino acids from CD44.

The peptide may, however, comprise amino acid sequences derived from other proteins. Therefore, the peptide compound of the invention includes heterologous fusion peptides consisting of (a) or (b) fused to a heterologous amino acid sequence. The heterologous amino acid sequence may comprise or consist of 1, 2, 3, 4 or more amino acids. The heterologous amino acid sequence may for example comprise or consist of at least 5 or at least 10 or at least 20 heterologous amino acids. The heterologous amino acids may be fused to the N- and/or C-terminus of the CD44-derived sequence.

The term "derivative" is intended to include variants and chemical derivatives of the peptides. A "variant" of a peptide is meant to refer to a molecule substantially similar to either the entire peptide, or a fragment thereof. A variant of a first peptide may be a second peptide which has 1 to 5, preferably 1 to 4, more preferably 1 to 3, more preferably 1 or 2 amino acid substitutions, additions and/or deletions with respect to said first peptide. For example, variants of SEQ ID NO:2 may have 1 to 5 amino acid substitutions with respect to SEQ ID NO:2, as long as the activity of inhibiting the complex formation between Met, HGF and CD44 leading to an inhibition of HGF mediated activation of ERK, is substantially the same as that of the peptide consisting of the amino acid sequence as shown in SEQ ID NO:2.

A molecule is said to be a "chemical derivative" of a first peptide when it contains additional chemical moieties not present in the first peptide. Such moieties may improve the molecule's solubility, absorption, biological half-life, etc. The moieties may alternatively decrease the toxicity of the molecule, eliminate or attenuate any undesirable side effect of the molecule, etc.

Generally, the derivative has at least 75%, pereferably at least 100% of the "activity of inhibiting the complex formation between Met, HGF and CD44 leading to an inhibition of HGF-mediated activation of ERK" of the first peptide from which it is derived.

It is preferred that the peptide of the invention is an isolated peptide. The term "isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

It is also preferred that the peptide of the invention is in a pure state. Preferably, the peptide is ≥ 80% pure, preferably ≥ 90% pure, more preferably ≥ 95% pure, even more preferably ≥ 99% pure and particularly preferred is a pharmaceutically pure state that is greater than 99.9% pure with respect to contaminating macromolecules, particularly other peptides. It is preferred that the peptide is free of infectious and pyrogenic agents. Preferably, a purified peptide is substantially free of other peptides. When used in this context, the term "pure" does not exclude the presence of the same peptide in alternative physical forms, such as dimers.

The peptides of the invention may be prepared by chemical synthesis or by recombinant expression in host cells. The preparation by chemical synthesis is preferred. As protein products, compounds of SEQ ID NO: 1 or 2 or other peptides of the present invention are amenable to production by the technique of solution- or solid-phase peptide synthesis. The synthetic peptide synthesis approach generally entails the use of automated synthesizers and appropriate resin as solid phase, to which is attached the C-terminal amino acid of the desired peptide. Extension of the peptide in the N-terminal direction is then achieved by successively coupling a suitably protected form of the next desired amino acid, using either FMOC- or BOC-based chemical protocols typically, until synthesis is complete. Protecting groups are then cleaved from the peptide, usually simultaneously with cleavage of peptide from the resin, and the peptide is then isolated and purified using conventional techniques, such as by reversed phase HPLC using acetonitrile as solvent and tri-fluoroacetic acid as ion-pairing agent. Such procedures are generally described in numerous publications and reference may be made, for example, to Stewart and Young, "Solid Phase Peptide Synthesis," 2nd Edition, Pierce Chemical Company, Rockford, III. (1984).
The invention therefore relates to a method for preparing a peptide described herein, comprising chemically synthesizing the peptide.

The invention further relates to a peptide compound of the invention for therapeutic use.

Another aspect of the invention is a pharmaceutical composition comprising a peptide compound described herein. The pharmaceutical composition preferably comprises a pharmaceutically acceptable carrier, excipient and/or diluent. Preferred embodiments of the pharmaceutical composition are described infra.

Another aspect of the invention is the use of a peptide compound of the invention for the manufacture of a medicament for the treatment and/or prevention of a disease in which the inhibition of abnormal growth factor receptor signaling has a beneficial effect. The disease to be treated and/or prevented can be any disease described infra in more detail.

Usually, the abnormal growth factor receptor signaling in which CD44 is functionally involved is selected from MET, FGF-R, PDGF-R, ErbB2, EGF-R, N-cadherin, N-CAM, Osteopontin and integrins. Preferably, the abnormal growth factor receptor signaling involves the complex formation of CD44, Met and HGF.

In one embodiment, the treatment or prevention of the disease can be a combination treatment. The treatment or prevention may further comprise a treatment selected from irradiation therapy, treatment with differentiation inducing drugs, treatment with chemotherapeutic drugs, cytostatic drugs, metabolism modifying drugs, treatment with cytotoxic drugs, hormone and anti-hormone therapy, immunotherapy, anti-angiogenic therapy and/or gene therapy.

The invention further relates to a method of screening for a compound capable of inhibiting complex formation of CD44, Met and HGF, comprising
(i) modifying a peptide compound of the invention; and
(ii) determining the inhibitory activity of the modified peptide.

Step (i) preferably comprises chemically modifying the peptide. The method may further comprise the step of selecting the modified peptide if the inhibitory activity of the modified peptide is greater than that of the peptide (prior to modification).

### DETAILED DESCRIPTION OF THE INVENTION

### USES

The invention provides a pharmaceutical composition comprising a compound of Formula (I) in free form or in the form of pharmaceutically acceptable salts and physiologically functional derivatives of the amino acid sequence depicted in Formula (I), together with a pharmaceutically acceptable diluent or carrier therefore.
**Formula (I): KEKWFENEWQGKNP** (SEQ ID NO:1)

Further described herein is a method for the treatment or prophylaxis of a condition where there is an advantage in inhibiting the formation of a complex consisting of CD44, c-met and HGF leading to an inhibition of HGF-mediated activation of ERK which comprises the administration of an effective amount of a compound of Formula (I) and physiologically acceptable salts or physiologically functional derivatives thereof, such as indicated by their amino acid sequence in table 1 (the corresponding linker scanning - Is - regions are indicated), or derived from chemical synthesis diverted from these peptide sequences. Regions of Is7 and Is8 are underlined:

**Table 1**

| | | | | | | | | | | | | | | | | | | | | | | |
|---|---|---|---|---|---|---|---|---|---|---|---|---|---|---|---|---|---|---|---|---|---|---|
| **Is** | | | | | | | **6** | | | **7** | | | **8** | | | **9** | | | | | | |
| | | | | | K | E | K | W | F | E | N | E | W | Q | G | K | N | P | | | | |
| | | | | | | E | K | W | F | E | N | E | W | Q | G | K | N | P | | | | |
| | | | | | | E | K | W | F | E | N | E | W | Q | G | K | N | | | | | |
| | | | | | | E | K | W | F | E | N | E | W | Q | G | K | | | | | | |
| | | | | | | E | K | W | F | E | N | E | W | Q | G | | | | | | | |
| | | | | | | E | K | W | F | E | N | E | W | Q | | | | | | | | |
| | | | | | | E | K | W | F | E | N | E | W | | | | | | | | | |
| | | | | | | E | K | W | F | E | N | E | | | | | | | | | | |
| | | | | | | | K | W | F | E | N | E | W | Q | G | K | N | P | | | | |
| | | | | | | | K | W | F | E | N | E | W | Q | G | K | N | | | | | |
| | | | | | | | K | W | F | E | N | E | W | Q | G | K | | | | | | |
| | | | | | | | K | W | F | E | N | E | W | Q | G | | | | | | | |
| | | | | | | | K | W | F | E | N | E | W | Q | | | | | | | | |
| | | | | | | | K | W | F | E | N | E | W | | | | | | | | | |
| | | | | | | | K | W | F | E | N | E | | | | | | | | | | |
| | | | | | | | | W | F | E | N | E | W | Q | G | K | N | P | | | | |
| | | | | | | | | W | F | E | N | E | W | Q | G | K | N | | | | | |
| | | | | | | | | W | F | E | N | E | W | Q | G | K | | | | | | |
| | | | | | | | | W | F | E | N | E | W | Q | G | | | | | | | |
| | | | | | | | | W | F | E | N | E | W | Q | | | | | | | | |
| | | | | | | | | W | F | E | N | E | W | | | | | | | | | |
| | | | | | | | | W | F | E | N | E | | | | | | | | | | |
| | | | | | | | | | F | E | N | E | W | Q | G | K | N | P | | | | |
| | | | | | | | | | F | E | N | E | W | Q | G | K | N | | | | | |
| | | | | | | | | | F | E | N | E | W | Q | G | K | | | | | | |
| | | | | | | | | | F | E | N | E | W | Q | G | | | | | | | |
| | | | | | | | | | F | E | N | E | W | Q | | | | | | | | |
| | | | | | | | | | F | E | N | E | W | | | | | | | | | |
| | | | | | | | | | F | E | N | E | | | | | | | | | | |
| | | | | | | | | | | E | N | E | W | Q | G | K | N | P | | | | |
| | | | | | | | | | | E | N | E | W | Q | G | K | N | | | | | |
| | | | | | | | | | | E | N | E | W | Q | G | K | | | | | | |
| | | | | | | | | | | E | N | E | W | Q | G | | | | | | | |
| | | | | | | | | | | E | N | E | W | Q | | | | | | | | |
| | | | | | | | | | | E | N | E | W | | | | | | | | | |
| | | | | | | | | | | E | N | E | | | | | | | | | | |
| | | | | | | | | | | | N | E | W | Q | G | K | N | P | | | | |
| | | | | | | | | | | | N | E | W | Q | G | K | N | | | | | |
| | | | | | | | | | | | N | E | W | Q | G | K | | | | | | |
| | | | | | | | | | | | N | E | W | Q | G | | | | | | | |
| | | | | | | | | | | | N | E | W | Q | | | | | | | | |
| | | | | | | | | | | | N | E | W | | | | | | | | | |
| | | | | | | | | | | | N | E | | | | | | | | | | |
| is | | | | | | | **6** | | | **7** | | | **8** | | | **9** | | | | | | |

Further described herein is the use of compounds of the Formula (I) and of their pharmacologically tolerable salts or physiologically functional derivatives for the production of a medicament for the prevention and treatment of diseases, where inhibition of the formation of a complex consisting of CD44, c-met and HGF leading to an inhibition of HGF-mediated activation of ERK is of benefit.

In another embodiment of the invention, compounds derived from the corresponding amino acid sequences of the human CD44 v6 region are included, based on the strong homology between the rat sequence - as used in Formula (I) - and human sequence, as depicted in the following figure also displaying the sequence accession numbers:

Thus, also the following compounds based on the human CD44 v6 amino acid sequences, derived from the corresponding table 2 below, are included in this invention and are depicted according to Formula (II):
**Formula (II): K E Q W F G N R W H E G Y R** (SEQ ID NO:2)

Further described herein is a method for the treatment or prophylaxis of a condition where there is an advantage in inhibiting the formation of a complex consisting of CD44, c-met and HGF leading to an inhibition of HGF-mediated activation of ERK which comprises the administration of an effective amount of a compound of Formula (II) and physiologically acceptable salts or physiologically functional derivatives thereof, such as indicated by their amino acid sequence in table 2 (the corresponding linker scanning regions are indicated), or derived from chemical synthesis diverted from these peptide sequences. The human CD44 v6 regions corresponding to ls7 and Is8 are underlined:

**Table 2**

| | | | | | | | | | | | | | | | | | | | | | |
|---|---|---|---|---|---|---|---|---|---|---|---|---|---|---|---|---|---|---|---|---|---|
| is | | | | | | | **6** | | | **7** | | | **8** | | | **9** | | | | | |
| | | | | | K | E | Q | W | F | G | N | R | W | H | E | G | Y | R | | | |
| | | | | | | E | Q | W | F | G | N | R | W | H | E | G | Y | R | | | |
| | | | | | | E | Q | W | F | G | N | R | W | H | E | G | Y | | | | |
| | | | | | | E | Q | W | F | G | N | R | W | H | E | G | | | | | |
| | | | | | | E | Q | W | F | G | N | R | W | H | E | | | | | | |
| | | | | | | E | Q | W | F | G | N | R | W | H | | | | | | | |
| | | | | | | E | Q | W | F | G | N | R | W | | | | | | | | |
| | | | | | | | Q | W | F | G | N | R | | | | | | | | | |
| | | | | | | | Q | W | F | G | N | R | W | H | E | G | Y | R | | | |
| | | | | | | | Q | W | F | G | N | R | W | H | E | G | Y | | | | |
| | | | | | | | Q | W | F | G | N | R | W | H | E | G | | | | | |
| | | | | | | | Q | W | F | G | N | R | W | H | E | | | | | | |
| | | | | | | | Q | W | F | G | N | R | W | H | | | | | | | |
| | | | | | | | Q | W | F | G | N | R | W | | | | | | | | |
| | | | | | | | Q | W | F | G | N | R | | | | | | | | | |
| | | | | | | | | W | F | G | N | R | W | H | E | G | Y | R | | | |
| | | | | | | | | W | F | G | N | R | W | H | E | G | Y | | | | |
| | | | | | | | | W | F | G | N | R | W | H | | | | | | | |
| | | | | | | | | W | F | G | N | R | W | H | E | | | | | | |
| | | | | | | | | W | F | G | N | R | W | H | | | | | | | |
| | | | | | | | | W | F | G | N | R | W | | | | | | | | |
| | | | | | | | | W | F | G | N | R | | | | | | | | | |
| | | | | | | | | | F | G | N | R | W | H | E | G | Y | R | | | |
| | | | | | | | | | F | G | N | R | W | H | E | G | Y | | | | |
| | | | | | | | | | F | G | N | R | W | H | E | G | | | | | |
| | | | | | | | | | F | G | N | R | W | H | E | | | | | | |
| | | | | | | | | | F | G | N | R | W | H | | | | | | | |
| | | | | | | | | | F | G | N | R | W | | | | | | | | |
| | | | | | | | | | F | G | N | R | | | | | | | | | |
| | | | | | | | | | | G | N | R | W | H | E | G | Y | R | | | |
| | | | | | | | | | | G | N | R | W | H | E | G | Y | | | | |
| | | | | | | | | | | G | N | R | W | H | E | G | | | | | |
| | | | | | | | | | | G | N | R | W | H | E | | | | | | |
| | | | | | | | | | | G | N | R | W | H | | | | | | | |
| | | | | | | | | | | G | N | R | W | | | | | | | | |
| | | | | | | | | | | G | N | R | | | | | | | | | |
| | | | | | | | | | | | N | R | W | H | E | G | Y | R | | | |
| | | | | | | | | | | | N | R | W | H | E | G | Y | | | | |
| | | | | | | | | | | | N | R | W | H | E | G | | | | | |
| | | | | | | | | | | | N | R | W | H | E | | | | | | |
| | | | | | | | | | | | N | R | W | H | | | | | | | |
| | | | | | | | | | | | N | R | W | | | | | | | | |
| | | | | | | | | | | | N | R | | | | | | | | | |
| is | | | | | | | **6** | | | **7** | | | **8** | | | **9** | | | | | |

The compounds of Formula (I) and Formula (II) according to the invention can form salts with inorganic or organic acids or bases. Examples of such salts are, for example, alkali metal salts, in particular sodium and potassium salts, or ammonium salts.

Further disclosed therein are compounds and medicaments prepared therewith that are generally useful to induce growth arrest and/or differentiation and/or apoptosis of cells such as hyperproliferative cells, invasive cells, tumor cells and/or inhibit or reduce their invasive potential. The therapeutic effect of the invention may arise through one or more mechanisms, including but not limited to, the regulation of cell proliferation, cell activation, cell survival, cell differentiation, cell cycle, cell maturation, cell migration, cell adhesion, cell invasion and cell death or to induce systemic changes in metabolism such as changes in sugar, lipid or protein metabolism, the inhibition of new blood vessel formation (anti-angiogenesis), the inhibition of tumor spread into other organs (anti-metastatic), the inhibition of tumor spread into neighbouring normal structures (anti-invasive) or the promotion of programmed cell death (apoptosis).

Further disclosed therein are compounds and their use for the manufacture of medicaments that are also suitable for the treatment or prevention of diseases in which the inhibition of the formation of a complex consisting of CD44, c-met and HGF, or of HGF-mediated signaling activity has a beneficial effect resulting in reprogramming or killing of, e.g., a patient's tumor or diseased cells and thus causing a clinical improvement of the patient's condition. In addition, also the formation of complexes of CD44 involving other growth factor recetors, such as, but not limited to, FGFR, PDGFR, ErbB2, EGFR, Osteopontin, and integrins, followed by a CD44 mediated signaling of these growth factor receptors, so that an inhibition of the formation of such complexes might be therapeutically benefical in diseases in which the formation of such complexes contributes to the disease onset, manifestation or progression. Examples of such diseases comprise, e.g., hyperproliferative disorders, diseases associated with an increased invasive potential of cells, cancer and inflammatory disorders and include, but are not limited to, estrogen receptor-dependent breast cancer, estrogen receptor-independent breast cancer, hormone receptor-dependent prostate cancer, hormone receptor-independent prostate cancer, brain cancer, renal cancer, colon cancer, familial adenomatous polyposis (FAP), colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity. Treatment with compounds of the present invention is particularly also useful for treating tumor metastases.

Application of the compounds may be systemic, locally applied, such as intratumorally, or topically. Additionally, the invention may also be beneficial by reverting inappropriate gene expression in diseases based on aberrant formation of a complex consisting of CD44, c-met and HGF, or abberant of HGF-mediated signaling activity, such as conditions associated with abnormal gene expression, such as inflammatory disorders (including but not limited to inflammatory disorders of the skin), diabetes, thalassemia, cirrhosis, protozoal infection, or the like and all types of autoimmune diseases, in particular rheumatoid arthritis, rheumatoid spondylitis, all forms of rheumatism, osteoarthritis, gouty arthritis, multiple sclerosis, insulin dependent diabetes mellitus and non-insulin dependent diabetes, asthma, rhinitis, uveithis, lupus erythematoidis, ulcerative colitis, Morbus Crohn, inflammatory bowel disease, as well as other chronic inflammations, chronic diarrhea, as well as disorders of the skin, such as acne, atopic dermatitis, psoriasis, actinic keratosis and other hyperproliferative skin disorders.

Further disclosed herein is a diagnostic method to identify tumors comprising the step of testing in vitro whether a tumor is responsive to treatment either with compounds or derivatives of Formula (I) or Formula (II) alone or used in combination with established tumor therapeutics. The method preferably comprises the method for the identification of genes regulated by these treatments. In a particular embodiment, the diagnostic method comprises the use of nucleic acid technology, preferably of hybridization or polymerase chain reaction for detection. Other types of nucleic acid technology, however, may be employed. In another embodiment the method comprises the use of specific antibodies against differentially regulated proteins for detection. For this purpose proteins encoded by the genes showing deregulation of their expression upon treatment using compounds or derivatives of Formula (I) or Formula (II), would be expressed e.g. in recombinant expression systems and antibodies directed against these proteins would be generated. Subsequently such antibodies could be used (or patterns of antibodies) to characterize the status of a diseased cell or tissue, e.g., of a tumor or tumor cells for diagnostic and/or prognostic reasons.

In general, the present invention provides novel possibilities to treat various human diseases. The inhibition of the formation of a complex consisting of CD44, c-met and HGF, or of HGF-mediated signaling activity with compounds of Formula (I) or Formula (II) may be used in combination with clinically established therapeutic drugs for the treatment of, e.g., cancers of different origins. Using these compounds in such combinatorial treatment is considered to generate superior therapeutic success in patients than the corresponding therapeutic drugs used on their own. Such combinatorial treatments could result in a decrease of the therapeutic doses of, e.g., chemotherapeutic reagents required, and could thus limit the partly very serious side effects of frequently used therapies.

Further aspects of the present invention comprise the combination of compounds of Formula (I) and Formula (II) with, but not restricted to, therapeutic principles currently in clinical use or in clinical development, such as
- Chemotherapeutic or cytotoxic drugs (e.g., 5-FU, taxol, cisPlatinum, camptothecin, gemcitabine, doxorubicine, irinothecan)
- Inhibitors of histone modifying enzymes, such as histone acetylases and deacetylases, histone methylases, histone sumoylating enzymes
- Differentiation inducing drugs (e.g., vitamin D, retinoic acid, cytokines such as 11-3, II-6, SCF, G-CSF, GM-CSF, TNF)
- Radiation therapy (e.g., x-rays or gamma rays)
- Immunological approaches (antibody therapy, vaccination)
- Combined immunotherapeutic/cytotoxic approaches (e.g., antibodies conjugated with cytotoxic components)
- Anti-angiogenesis approaches
- Metabolism regulating drugs

### FORMULATION

The compounds and salts thereof can be formulated as pharmaceutical compositions (e.g., liquids, suspensions, emulsions, lozenges, cachets, ampoules, suppositories, pessaries, ointments, gels, pastes, sprays, lotions, oils, boluses, electuaries, aerosols, powders, granules, tablets, pills, capsules, injections, solutions, foams, creams, enemas and the like) comprising at least one such compound alone or in admixture with pharmaceutically acceptable carriers, excipients and/or diluents. The pharmaceutical compositions can be formulated in accordance with a conventional method.

### DOSAGE

Specific dose levels for any particular patient will be employed depending upon a variety of factors including the age, body weight, general health, sex, diet, and prior medication, and the severity of the particular disease of the patient, and the activity of specific compounds employed, time of administration, route of administration, rate of excretion, the duration of the treatment, other drugs, compounds, and/or materials used in combination. It will be appreciated that the appropriate dosage of the active compounds, and compositions comprising the active compounds, can vary from patient to patient. Determining the optimal dosage will generally involve balancing of the level of therapeutic benefit against any risk or deleterious side effects of the treatments of the present invention.

Administration in vivo can be effected in one dose, continuously or intermittently throughout the course of treatment. Methods of determining the most effective means and dosages of administration are well known to those of skill in the art, and will vary with the formulation used for therapy, the purpose of the therapy, the target cell being treated, and the subject being treated. Single or multiple administrations can be carried out with the dose level and pattern being selected by the treating physician.

In general, a suitable systemic dose of the active compound is in the range of about 0.01 to about 1000 mg per kilogram body weight preferably 0.1 to 500 mg per kilogram body weight and even more preferably 1.0 to 500 mg per kilogram body weight of the subject per day. Where the active ingredient is a salt, an ester, or the like, the amount administered is calculated on the basis the parent compound and so the actual weight to be used is increased proportionately.
In case the compound is applied locally, e.g., intratumorally or topically on skin, the amount of compound may vary from the above given estimation. However, such an application would aim at reaching local concentrations of the drug, e.g. intratumoral concentration, ranging from about 0.1ng/ml to 10 mg/ml, more preferred from 1 ng/ml to 1 mg/ml.
The various embodiments described in this application can be combined with each other.
**Figure 1** shows the functional analysis using linker scan (Is) mutations of the CD44 v6 region (Example 1).
**Figure 2** shows the functional analysis of HGF signaling in dependency of soluble CD44 v6 expression. (Example 1).
**Figure 3** shows functional competition studies using CD44v6 derived peptides (Example 3).
**Figure 4** shows functional competition studies using further rat CD44v6 derived peptides to specify the peptide regions required for competitive activities (Example 3).
**Figure 5** shows functional competition studies using human CD44v6 derived peptides to specify the peptide regions required for competitive activities in human 293T cells (Example 4).
**Figure 6** shows functional competition studies using human CD44v6 derived peptides to specify the peptide regions required for competitive activities in human HT29 carcinoma cells (Example 4).

The following examples further illustrate the invention:

### Example 1

### Met-Signaling in the presence of CD44 Mutants (Linker-Scanning-Mutants in the v6 region)

### Method:

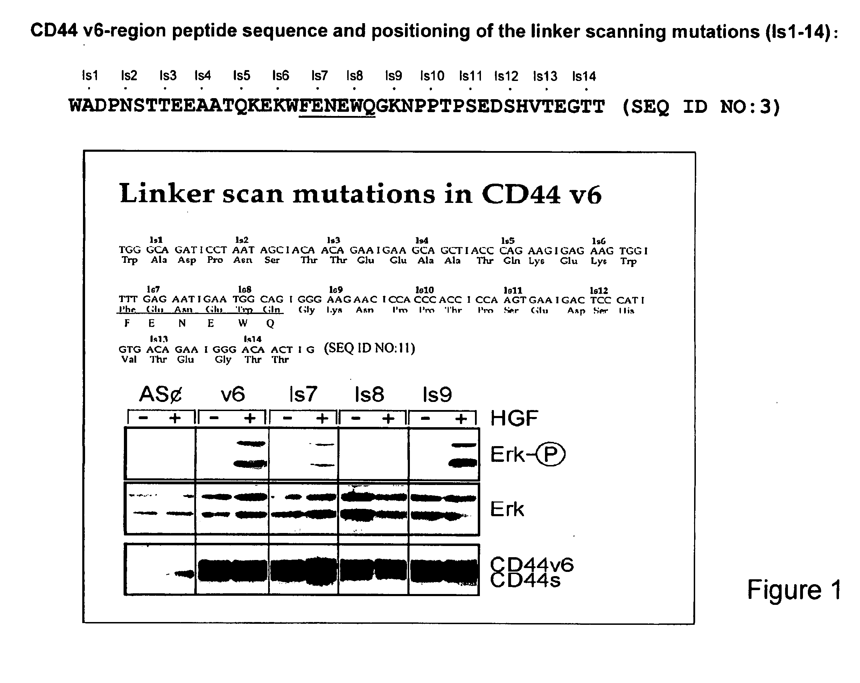

The rat pancreatic carcinoma cell line BSp73AS10, expressing the standard form of CD44 (CD44s), was stably transfected with plasmids coding for CD44 variants containing the v6 exon (CD44v). In these CD44 variants, exon v6 was present either as wt sequence (v6) or
as a mutant sequence constructed by linker scan (Is) mutations in the regions indicated (ls1-ls14). Cells were seeded in a 24-well plate of 10⁵ cells per well, next day cells were starved for 24 h in serum-free medium and then induced with 10 ng/ml HGF for 5 min.
Lysis was done in Laemmli Sample buffer. Equal amounts of Lysate were subjected to denaturing gel electrophoresis and Western blotting. Filters were probed as indicated with antibodies against ERK (Santa Cruz) (Figure 1, middle blot panel), against phosphorylated ERK, ERK-P (NEB) (Figure 1, upper blot panel), or CD44 constant region (IM7, Pharmingen) (Figure 1, lowest blot panel). The results should indicate whether HGF caused c-Met and c-Met-dependent ERK phosporylation.

### Results:

Figure 1 shows the result of the functional analysis using linker scan mutations of the CD44 v6 region. An intact v6 region (v6) is capable of mediating HGF induced signaling, measured by estimation of activated, i.e., phosphorylated ERK protein (ERK-P). Mutations designated as Is7 and Is8 clearly reduce (Is7) or even almost abolish, i.e., an inhibition of more than 90% (Is8) this activity of HGF signaling, whereas other mutations, e.g., Is9, do not result in such an inhibition of ERK activation. This finding implies that v6 peptide regions, spanning the amino acid sequence containing the Is7- and the ls8-mutation are crucial for HGF signalling (regions of ls7 and Is 8 are underlined).

### Example 2

### Met-Signaling in the presence of CD44 v6 and soluble CD44

### Method:

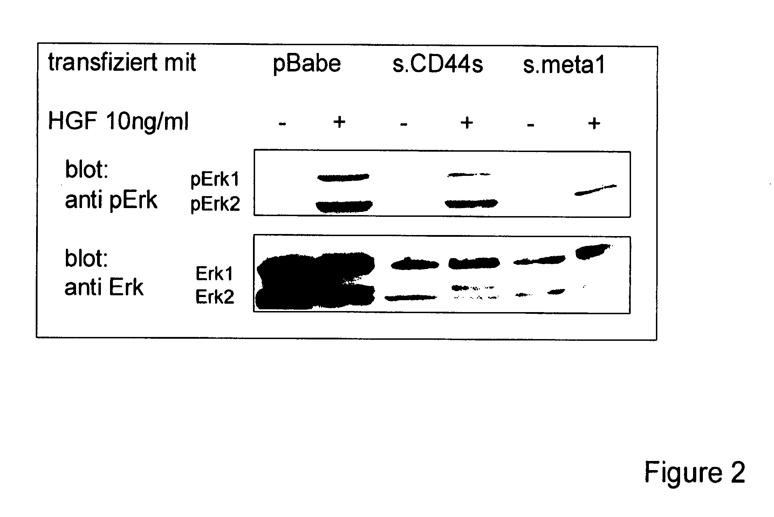

The rat pancreatic carcinoma cell line BSp73AS10, expressing the standard form of CD44 (CD44s), was stably transfected with a plasmid coding for CD44v6 to establish conditions that would allow Met activation. These stable transfectants were transiently infected with plasmids coding for soluble standard CD44 (s.CD44s) or soluble CD44v6 (s.meta1) (soluble means that the transmembrane and cytoplasmic portions of CD44 were deleted and thus the protein was secreted by the cells) or empty vector, conferring just puromycin resistance (pBabe). After transfection, cells were selected for transient transfectants using 2 µg/ml puromycin in serum-free medium for 24h. After that supernatant was cleared by centrifugation, supplemented with 10 ng/ml HGF and re-added to cells for 5 min. Lysis was done in Laemmli Sample buffer. Equal amounts of Lysate were subjected to denaturing gel electrophoresis and Western blotting. Filters were probed as indicated with antibodies directed against ERK (Santa Cruz), or with antibodies specific for phosphorylated and thus activated ERK, pERK (NEB), as depicted in figure 2.

### Results:

In figure 2 results from a functional analysis of HGF signaling in dependency of soluble CD44 v6 expression is presented. Only with the expression of soluble CD44 v6 protein (s.meta1) a competition for free HGF can be achieved which results in the reduced activation of HGF-mediated phosphorylation of ERK.

### Example 3

### Met-Signaling in the presence of CD44v6 and CD44v6-peptides

### Method:

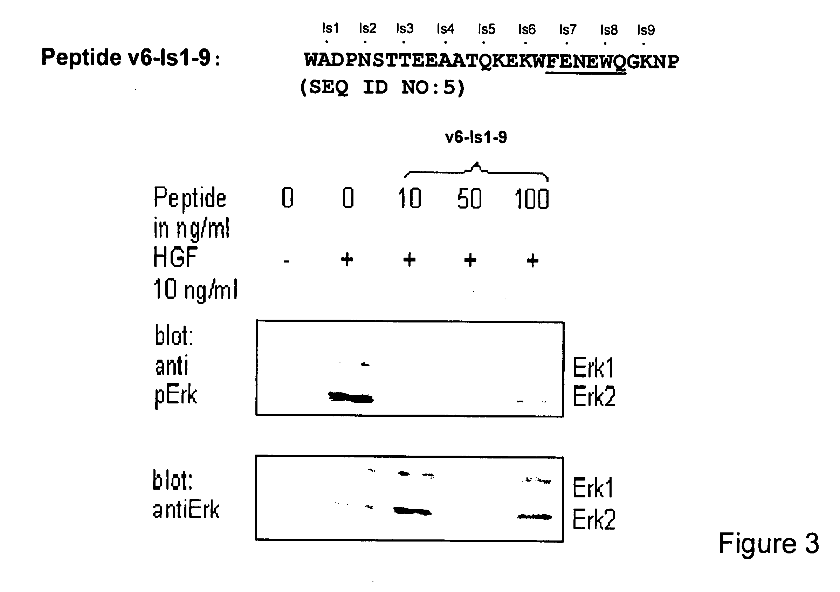

In a competition study experiment, depicted in figure 3, the following rat CD44v6 derived peptide containing the Is1-9 region has been used:

The rat pancreatic carcinoma cell line BSp73AS10, expressing the standard form of CD44 (CD44s), was stably transfected with a plasmid coding for CD44v6. These cells were seeded in a 24-well plate of 10⁵ cells per well. On the next day cells were starved for 24 h in serum free medium, then incubated with 10, 50 and 100 ng/ml peptide v6-ls1-9 for 5 min as indicated and subsequently induced with 10 ng/ml HGF for 5 min.
Lysis was done in Laemmli Sample buffer. Equal amounts of Lysate were subjected to denaturing gel electrophoresis and Western blotting. Filters were probed as indicated with antibodies directed against ERK (Santa Cruz), or with antibodies specific for phosphorylated ERK, pERK (NEB), as depicted in figure 3.

### Results:

Figure 3 shows results from this in vitro competition assay. The peptide which has been designated **v6-Is1-9** competes for the v6 sequence of the endogenous CD44 variant and thus, is capable of competing out HGF-derived signaling as measured by activation, i.e., phosphorylation of ERK which is reduced by more than 50%. This implies that peptide sequence motifs present in this peptid are capable of interfering with the formation of the trimeric complex of CD44, HGF and c-Met. The amino acid sequence of this v6-Is1-9 peptide contains the region that also harbors the location of the mutations Is7 and Is8 (regions of Is7 and Is 8 are underlined below) that have been identified in figure 1 as being crucial for the formation of the trimeric complex and the HGF mediated signaling into the ERK pathway.

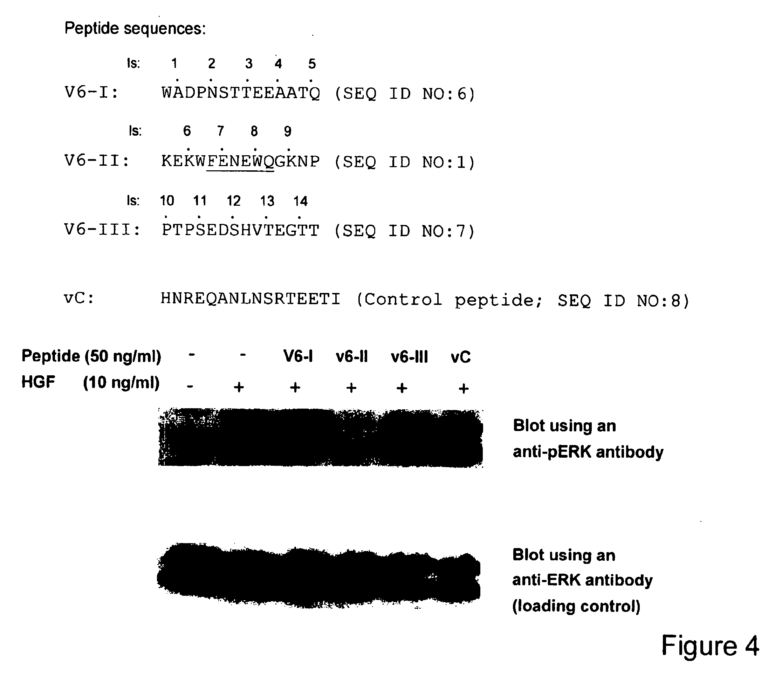

To restrict the peptide regions required for such competitive activities further, the experiments depicted in figure 4 extended this analysis to a series of additional, shorter peptides. For this purpose the following rat peptide sequences have been used, shown here with the locations of the corresponding linker scanning (Is1-14) sites:

### Method:

The rat pancreatic carcinoma cell line BSp73AS10, expressing the standard form of CD44 (CD44s), was stably transfected with a plasmid coding for CD44v6. These cells were seeded in a 24-well plate of 1.5 x 10⁵ cells per well. On the next day cells were starved for 24 h in serum free medium, then incubated with 50 ng/ml of the peptides (Figure 4) for 5 min as indicated and subsequently induced with 10 ng/ml HGF for 5 min at 37° C.
Lysis was done in Laemmli Sample buffer. Equal amounts of Lysate were subjected to denaturing gel electrophoresis and Western blotting. Filters were probed as indicated with antibodies directed against ERK (Santa Cruz), or with antibodies specific for phosphorylated ERK, pERK (NEB), as depicted in figure 4.

### Results:

Figure 4 shows results from these in vitro competition assays. From the peptides analysed, only the peptide designated **v6-II** competes effectively for the v6 sequence of the endogenous CD44 variant and thus, is capable of competing out HGF-derived signaling as measured by activation, i.e., phosphorylation of ERK which is reduced by 75-95%. This implies that peptide sequence motifs present in this peptid are especially capable of interfering with the formation of the trimeric complex of CD44, HGF and c-Met. Again, the amino acid sequence of this **v6-II** peptide contains the region that harbors the location of the mutations Is7 and Is8 (regions of Is7 and Is 8 are underlined, see above) that have also been identified in the previously described experiments as being crucial for the formation of the trimeric complex and the HGF mediated signaling into the ERK pathway.

To verify these data derived with the rat CD44 system for the human CD44 system, and to identify the required human peptide sequences which interfere with HGF mediated activation of the ERK pathway, corresponding studies were performed using human CD44v6 peptides for competition assays in Example 4 as already employed and decribed herein.

### Example 4

### Erk-Signaling induced by HGF can be inhibited by human CD44v6-peptides

### Method:

To identify the human sequences corresponding to the rat v6 peptides the rat and the human CD44 protein containing exon v6 sequences were taken from the database and aligned by Clustal W Method. The resulting aligned comparisson of the rat and the human derived CD44v6 peptides is shown below:

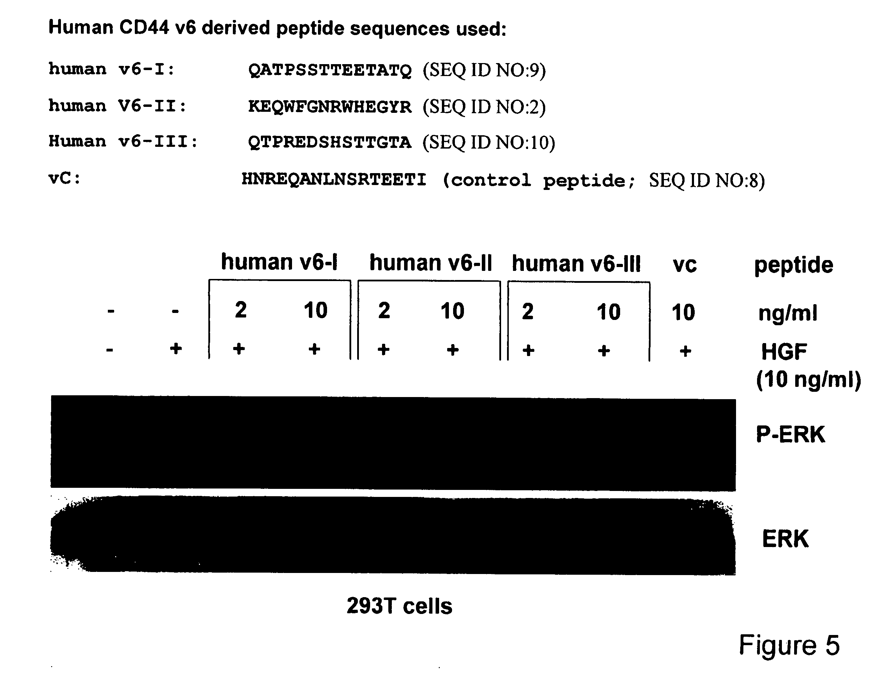

In the employed peptide competition study experiments, depicted in figure 5, the following CD44v6 derived human peptides and a control peptide have been used:

| | |
|---|---|
| human v6-I: | QATPSSTTEETATQ (SEQ ID NO:9) |
| human v6-II: | KEQWFGNRWHEGYR (SEQ ID NO:2) |
| human v6-III: | QTPREDSHSTTGTA (SEQ ID NO:10) |
| vC: | HNREQANLNSRTEETI (control peptide; SEQ ID NO:8) |

The human embryonal kidney cell line HEK-293T, expressing the standard form of CD44 (CD44s) and several variant isoforms of CD44 was used. These cells were seeded in a 24-well plate of 10⁵ cells per well. On the next day cells were starved for 24 h in serum free medium, then incubated with 2 and 10 ng/ml peptide for 5 min as indicated and subsequently induced with 10 ng/ml HGF for 5 min.

Lysis was done in Laemmli Sample buffer. Equal amounts of Lysate were subjected to denaturing gel electrophoresis and Western blotting. Filters were probed as indicated with antibodies directed against ERK (Santa Cruz), or with antibodies specific for phosphorylated ERK, pERK (NEB), as depicted in figure 5.

### Results:

Figure 5 shows results from these in vitro competition assays. From the peptides analysed, only the peptide designated **human v6-II** competes effectively for the v6 sequence of the endogenous CD44 variant and thus, is capable of competing out HGF-derived signaling as measured by activation, i.e., phosphorylation of ERK which is reduced by at least 30-50%. This implies that peptide sequence motifs present in this peptid are especially capable of interfering with the formation of the trimeric complex of CD44, HGF and c-Met and that the CD44 peptide sequences involved in the complex formation in the rat and and the human system are homologous. To verify this further it was analysed if the human v6-II peptide is able to disrupt HGF signaling in a human carcinoma cell line.

### Method:

The human colon carcinoma cell line HT29, expressing the standard form of CD44 (CD44s) and several variant isoforms of CD44 were used for the peptide competition study experiment. The following CD44v6 derived human peptides have been used:

| | |
|---|---|
| human v6-I: | QATPSSTTEETATQ (SEQ ID NO:9) |
| human v6-II: | KEQWFGNRWHEGYR (SEQ ID NO:2) |
| human v6-III: | QTPREDSHSTTGTA (SEQ ID NO:10) |

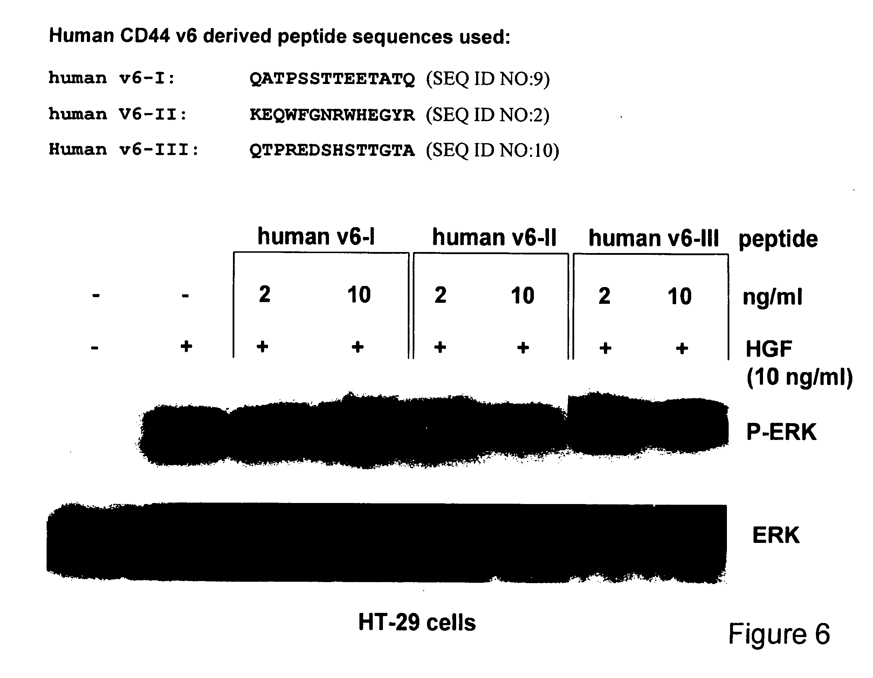

Cells were seeded in a 24-well plate of 10⁵ cells per well. On the next day cells were starved for 24 h in serum free medium, then incubated with 2 and 10 ng/ml peptide for 5 min as indicated and subsequently induced with 10 ng/ml HGF for 5 min.
Lysis was done in Laemmli Sample buffer. Equal amounts of Lysate were subjected to denaturing gel electrophoresis and Western blotting. Filters were probed as indicated with antibodies directed against ERK (Santa Cruz), or with antibodies specific for phosphorylated ERK, pERK (NEB), as depicted in figure 6.

### Results:

Figure 6 shows results from these in vitro competition assays. From the peptides analysed, only the peptide designated **human v6-II** competes effectively for the v6 sequence of the endogenous CD44 variant and thus, is capable of competing out HGF-derived signaling as measured by activation, i.e., phosphorylation, of ERK which is reduced by at least 30-50%. This implies that human v6-II peptides can be used to disrupt c-Met dependent signaling in human carcinoma cell lines.
Thus, such peptide motifs may directly serve as therapeutic compounds, or give rise to the structural development of novel drugs, that exert this signaling inhibitory activity, and their use for the prevention or treatment of human diseases, such as cancer or inflammatory disorders.

Thus, such peptide motifs may directly serve as therapeutic compounds, or give rise to the structural development of novel drugs, that exert this signaling inhibitory activity, and their use for the prevention or treatment of human diseases, such as hyperproliferative disorders, disorders based on an enhanced invasive potential of cells, cancer or inflammatory disorders.

## Claims

1. A peptide compound selected from the group consisting of
(a) peptides consisting of the amino acid sequence as shown in SEQ ID N0:1 or SEQ ID NO:2;
(b) peptides consisting of any one of the following amino acid sequences:
amino acids 2-14 of SEQ ID NO:1 or 2;
amino acids 2-13 of SEQ ID NO:1 or 2;
amino acids 2-12 of SEQ ID NO:1 or 2;
amino acids 2-11 of SEQ ID NO:1 or 2;
amino acids 3-14 of SEQ ID NO:1 or 2;
amino acids 3-13 of SEQ ID NO:1 or 2;
amino acids 3-12 of SEQ ID NO:1 or 2;
amino acids 3-11 of SEQ ID NO:1 or 2;
amino acids 4-14 of SEQ ID NO:1;
amino acids 4-13 of SEQ ID NO:1 or 2;
amino acids 4-12 of SEQ ID NO:1 or 2;
amino acids 4-11 of SEQ ID NO:1 or 2;
amino acids 5-14 of SEQ ID NO:1 or 2;
amino acids 5-13 of SEQ ID NO:1 or 2;
amino acids 5-12 of SEQ ID NO:1 or 2;
amino acids 5-11 of SEQ ID NO: or 2;
amino acids 6-14 of SEQ ID NO:1 or 2;
amino acids 6-13 of SEQ ID NO:1 or 2;
amino acids 6-12 of SEQ ID NO:1 or 2;
amino acids 6-11 of SEQ ID NO:1 or 2;
amino acids 7-14 of SEQ ID NO:1 or 2;
amino acids 7-13 of SEQ ID NO:1 or 2;
amino acids 7-12 of SEQ ID NO:1 or 2;
amino acids 7-11 of SEQ ID NO:1 or 2;
and
(c) heterologous fusion peptides consisting of (a) or (b) fused to a heterologous amino acid sequence.

2. A peptide compound according to claim 1 capable of inhibiting the formation of a complex consisting of Met, HGF and CD44 leading to an inhibition of HGF mediated activation of ERK in an in vitro assay.

3. A peptide compound according to claim 1 or 2, or a peptide consisting of amino acids 4-14 of SEQ ID NO:2 for therapeutic use.

4. A pharmaceutical composition comprising a peptide compound according to claim 1 or 2.

5. A pharmaceutical composition according to claim 4, further comprising a pharmaceutically acceptable carrier, excipient and/or diluent.

6. A pharmaceutical composition according to claim 4 or 5, which is formulated as liquid, suspension, emulsion, lozenge, cachet, ampoule, suppository, pessary, ointment, gel, paste, spray, lotion, oil, boluse, electuary, aerosol, powder, granule, tablet, pill, capsule, injection, solution, foam, cream or enema.

7. The use of a peptide compound according to claim 1 or 2, or of a peptide consisting of amino acids 4-14 of SEQ ID NO:2 for the manufacture of a medicament for the treatment and/or prevention of tumor metastases.

8. The use according to claim 7, wherein the treatment or prevention of the disease further comprises a treatment selected from irradiation therapy, treatment with differentiation inducing drugs, treatment with chemotherapeutic drugs, cytostatic drugs, metabolism regulating drugs, treatment with cytotoxic drugs, hormone and anti-hormone therapy, immunotherapy, anti-angiogenic therapy and/or gene therapy.

9. The use according to claim 7 or 8, wherein the peptide compound is a cyclopeptide or a pharmaceutically acceptable salt.

10. A method of screening for a compound capable of inhibiting complex formation of CD44, Met and HGF leading to inhibition of HGF mediated activation of ERK, comprising
(i) modifying a peptide compound according to claim 1 or 2; and
(ii) determining the inhibitory activity of the modified peptide.

11. A method according to claim 10, wherein step (i) comprises chemically modifying the peptide compound.

## Patentansprüche

1. Peptidverbindung, ausgewählt aus der Gruppe, bestehend aus
(a) Peptiden, die aus der in SEQ ID NR.: 1 oder SEQ ID NR.: 2 gezeigten Aminosäuresequenz bestehen;
(b) Peptiden, die aus einer der folgenden Aminosäuresequenzen bestehen:
Aminosäuren 2 - 14 von SEQ ID NR.: 1 oder 2;
Aminosäuren 2 - 13 von SEQ ID NR.: 1 oder 2;
Aminosäuren 2 - 12 von SEQ ID NR.: 1 oder 2;
Aminosäuren 2 - 11 von SEQ ID NR.: 1 oder 2;
Aminosäuren 3 - 14 von SEQ ID NR.: 1 oder 2;
Aminosäuren 3 - 13 von SEQ ID NR.: 1 oder 2;
Aminosäuren 3 - 12 von SEQ ID NR.: 1 oder 2;
Aminosäuren 3 - 11 von SEQ ID NR.: 1 oder 2;
Aminosäuren 4 - 14 von SEQ ID NR.: 1;
Aminosäuren 4 - 13 von SEQ ID NR.: 1 oder 2;
Aminosäuren 4 - 12 von SEQ ID NR.: 1 oder 2;
Aminosäuren 4 - 11 von SEQ ID NR.: 1 oder 2;
Aminosäuren 5 - 14 von SEQ ID NR.: 1 oder 2;
Aminosäuren 5 - 13 von SEQ ID NR.: 1 oder 2;
Aminosäuren 5 - 12 von SEQ ID NR.: 1 oder 2;
Aminosäuren 5 - 11 von SEQ ID NR.: 1 oder 2;
Aminosäuren 6 - 14 von SEQ ID NR.: 1 oder 2;
Aminosäuren 6 - 13 von SEQ ID NR.: 1 oder 2;
Aminosäuren 6 - 12 von SEQ ID NR.: 1 oder 2;
Aminosäuren 6 - 11 von SEQ ID NR.: 1 oder 2;
Aminosäuren 7 - 14 von SEQ ID NR.: 1 oder 2;
Aminosäuren 7 - 13 von SEQ ID NR.: 1 oder 2;
Aminosäuren 7 - 12 von SEQ ID NR.: 1 oder 2;
Aminosäuren 7 - 11 von SEQ ID NR.: 1 oder 2;
und
(c) heterologen Fusionspeptiden, bestehend aus (a) oder (b), fusioniert an eine heterologe Aminosäuresequenz.

2. Peptidverbindung nach Anspruch 1, welche die Bildung eines Komplexes, bestehend aus Met, HGF und CD44, der zu einer Inhibierung der HGF-vermittelten Aktivierung von ERK in einem In-vitro-Assay führt, inhibieren kann.

3. Peptidverbindung nach Anspruch 1 oder 2 oder ein Peptid, bestehend aus den Aminosäuren 4 - 14 von SEQ ID NR.: 2, zur therapeutischen Verwendung.

4. Pharmazeutische Zusammensetzung, umfassend eine Peptidverbindung nach Anspruch 1 oder 2.

5. Pharmazeutische Zusammensetzung nach Anspruch 4, die ferner einen pharmazeutisch akzeptablen Träger, Hilfsstoff und/oder ein pharmazeutisch akzeptables Verdünnungsmittel umfasst.

6. Pharmazeutische Zusammensetzung nach Anspruch 4 oder 5, formuliert als Flüssigkeit, Suspension, Emulsion, Pastille, Cachet, Ampulle, Zäpfchen, Pessar, Salbe, Gel, Paste, Spray, Lotion, Öl, Bolus, Latwerge, Aerosol, Pulver, Granulat, Tablette, Pille, Kapsel, Injektion, Lösung, Schaum, Creme oder Klistier.

7. Verwendung einer Peptidverbindung nach Anspruch 1 oder 2 oder eines Peptids, bestehend aus den Aminosäuren 4 - 14 von SEQ ID NR.: 2, zur Herstellung eines Medikaments zur Behandlung und/oder Vorbeugung von Tumormetastasen.

8. Verwendung nach Anspruch 7, wobei die Behandlung oder Vorbeugung der Krankheit ferner eine Behandlung, ausgewählt aus Strahlentherapie, Behandlung mit die Differenzierung induzierenden Arzneimitteln, Behandlung mit Chemotherapeutika, Zytostatika, den Stoffwechsel regulierenden Arzneimitteln, Behandlung mit zytotoxischen Arzneimitteln, Hormon- und Antihormontherapie, Immuntherapie, antiangiogener Therapie und/oder Gentherapie, umfasst.

9. Verwendung nach Anspruch 7 oder 8, wobei die Peptidverbindung ein Cyclopeptid oder ein pharmazeutisch akzeptables Salz ist.

10. Verfahren zum Screenen nach einer Verbindung, die die Komplexbildung von CD44, Met und HGF, die zur Inhibierung der HGF-vermittelten Aktivierung von ERK führt, inhibieren kann, umfassend
(i) das Modifizieren einer Peptidverbindung nach Anspruch 1 oder 2 und
(ii) das Bestimmen der Inhibierungsaktivität des modifizierten Peptids.

11. Verfahren nach Anspruch 10, wobei Schritt (i) das chemische Modifizieren der Peptidverbindung umfasst.

## Revendications

1. Composé peptidique sélectionné dans le groupe consistant en :
(a) des peptides consistant en la séquence d'acide aminé telle que montrée dans la SEQ ID NO : 1 ou la SEQ ID NO : 2 ;
(b) des peptides consistant en l'une quelconque des séquences d'acide aminé suivantes :
acides aminés 2 à 14 de la SEQ ID NO : 1 ou 2 ;
acides aminés 2 à 13 de la SEQ ID NO : 1 ou 2 ;
acides aminés 2 à 12 de la SEQ ID NO : 1 ou 2 ;
acides aminés 2 à 11 de la SEQ ID NO : 1 ou 2 ;
acides aminés 3 à 14 de la SEQ ID NO : 1 ou 2 ;
acides aminés 3 à 13 de la SEQ ID NO : 1 ou 2 ;
acides aminés 3 à 12 de la SEQ ID NO : 1 ou 2 ;
acides aminés 3 à 11 de la SEQ ID NO : 1 ou 2 ;
acides aminés 4 à 14 de la SEQ ID NO : 1 ;
acides aminés 4 à 13 de la SEQ ID NO : 1 ou 2 ;
acides aminés 4 à 12 de la SEQ ID NO : 1 ou 2 ;
acides aminés 4 à 11 de la SEQ ID NO : 1 ou 2 ;
acides aminés 5 à 14 de la SEQ ID NO : 1 ou 2 ;
acides aminés 5 à 13 de la SEQ ID NO : 1 ou 2 ;
acides aminés 5 à 12 de la SEQ ID NO : 1 ou 2 ;
acides aminés 5 à 11 de la SEQ ID NO : 1 ou 2 ;
acides aminés 6 à 14 de la SEQ ID NO : 1 ou 2 ;
acides aminés 6 à 13 de la SEQ ID NO : 1 ou 2 ;
acides aminés 6 à 12 de la SEQ ID NO : 1 ou 2 ;
acides aminés 6 à 11 de la SEQ ID NO : 1 ou 2 ;
acides aminés 7 à 14 de la SEQ ID NO : 1 ou 2 ;
acides aminés 7 à 13 de la SEQ ID NO : 1 ou 2 ;
acides aminés 7 à 12 de la SEQ ID NO : 1 ou 2 ;
acides aminés 7 à 11 de la SEQ ID NO : 1 ou 2 ;
et
(c) des peptides de fusion hétérologues consistant en (a) ou (b) fusionné à une séquence d'acide aminé hétérologue.

2. Composé peptidique selon la revendication 1 capable d'inhiber la formation d'un complexe consistant en Met, HGF et CD44 conduisant à l'inhibition d'une activation médiée par HGF de ERK dans un échantillon in vitro.

3. Composé peptidique selon la revendication 1 ou 2, ou peptide consistant en les acides aminés 4 à 14 de la SEQ ID NO : 2 pour un usage thérapeutique.

4. Composition pharmaceutique comprenant un composé peptidique selon la revendication 1 ou 2.

5. Composition pharmaceutique selon la revendication 4, comprenant en outre un vecteur, un excipient et/ou un diluent pharmaceutiquement acceptable.

6. Composition pharmaceutique selon la revendication 4 ou 5, qui est formulée en tant que liquide, suspension, émulsion, pastille, cachet, ampoule, suppositoire, pessaire, onguent, gel, pâte, spray, lotion, huile, bolus, électuaire, aérosol, poudre, granule, tablette, pilule, capsule, injection, solution, mousse, crème ou énéma.

7. Utilisation d'un composé peptidique selon la revendication1 ou 2, ou peptide consistant en les acides aminés 4 à 14 de la SEQ ID NO : 2 pour la fabrication d'un médicament pour le traitement et/ou la prévention des métastases tumorales.

8. Utilisation selon la revendication 7, dans laquelle le traitement ou le prévention de la maladie comprend en outre un traitement choisi parmi une thérapie par irradiation, un traitement avec des médicaments induisant une différentiation, un traitement avec des médicaments chimiothérapeutiques, des médicaments cytostatiques, des médicaments régulant le métabolisme, un traitement avec des médicaments cytotoxiques, une thérapie hormonale et anti-hormonale, une immunothérapie, une thérapie anti-angiogénique et/ou une thérapie génique.

9. Utilisation selon la revendication 7 ou 8, dans laquelle le composé peptidique est un cyclopeptide ou un sel pharmaceutiquement acceptable.

10. Procédé de dépistage pour un composé capable d'inhiber la formation complexe de DC44, Met et HGF conduisant à l'inhibition de l'activité médiée par HGF de ERK, comprenant :
(i) la modification d'un composé peptidique selon la revendication 1 ou 2 ; et
(i) la détermination de l'activité inhibitoire du peptide modifié.

11. Procédé selon la revendication 10, dans lequel l'étape (i) comprend la modification par voie chimique du composé peptidique.


### SEQUENCE LISTING

<110> G2M Cancer Drugs AG
   Forschungszentrum Karlsruhe GmbH
<120> Peptidic compounds and Derivatives thereof for the Treatment of Human Diseases Through Inhibition of Signaling via Growth Factors
<130> Novel Peptides
<160> 11
<170> PatentIn version 3.2
<210> 1
   <211> 14
   <212> PRT
   <213> rattus norvegicus
<400> 1
   Lys Glu Lys Trp Phe Glu Asn Glu Trp Gln Gly Lys Asn Pro
   1               5                   10
<210> 2
   <211> 14
   <212> PRT
   <213> homo sapiens
<400> 2
   Lys Glu Gln Trp Phe Gly Asn Arg Trp His Glu Gly Tyr Arg
   1 5 10
<210> 3
   <211> 42
   <212> PRT
   <213> rattus norvegicus
<400> 3
   Trp Ala Asp Pro Asn Ser Thr Thr Glu Glu Ala Ala Thr Gln Lys Glu
   1 5 10 15
   Lys Trp Phe Glu Asn Glu Trp Gln Gly Lys Asn Pro Pro Thr Pro Ser
   20 25 30
   Glu Asp Ser His Val Thr Glu Gly Thr Thr
   35 40
<210> 4
   <211> 42
   <212> PRT
   <213> homo sapiens
<400> 4
   Gln Ala Thr Pro Ser Ser Thr Thr Glu Glu Thr Ala Thr Gln Lys Glu
   1 5 10 15
   Gln Trp Phe Gly Asn Arg Trp His Glu Gly Tyr Arg Gln Thr Pro Arg
   20 25 30
   Glu Asp Ser His Ser Thr Thr Gly Thr Ala
   35 40
<210> 5
   <211> 28
   <212> PRT
   <213> rattus norvegicus
<400> 5
   Trp Ala Asp Pro Asn Ser Thr Thr Glu Glu Ala Ala Thr Gln Lys Glu
   1 5 10 15
   Lys Trp Phe Glu Asn Glu Trp Gln Gly Lys Asn Pro
   20 25
<210> 6
   <211> 14
   <212> PRT
   <213> rattus norvegicus
<400> 6
   Trp Ala Asp Pro Asn Ser Thr Thr Glu Glu Ala Ala Thr Gln
   1 5 10
<210> 7
   <211> 14
   <212> PRT
   <213> rattus norvegicus
<400> 7
   Pro Thr Pro Ser Glu Asp Ser His Val Thr Glu Gly Thr Thr
   1 5 10
<210> 8
   <211> 16
   <212> PRT
   <213> artificial sequence
<220>
   <223> control peptide
<400> 8
   His Asn Arg Glu Gln Ala Asn Leu Asn Ser Arg Thr Glu Glu Thr Ile
   1 5 10 15
<210> 9
   <211> 14
   <212> PRT
   <213> homo sapiens
<400> 9
   Gln Ala Thr Pro Ser Ser Thr Thr Glu Glu Thr Ala Thr Gln
   1 5 10
<210> 10
   <211> 14
   <212> PRT
   <213> homo sapiens
<400> 10
   Gln Thr Pro Arg Glu Asp Ser His Ser Thr Thr Gly Thr Ala
   1               5                   10
<210> 11
   <211> 127
   <212> DNA
   <213> rattus norvegicus
<400> 11
   tgggcagatc ctaatagcac aacagaagaa gcagctaccc agaaggagaa gtggtttgag    60
   aatgaatggc aggggaagaa cccacccacc ccaagtgaag actcccatgt gacagaaggg    120
   acaactg    127